(19) Europäisches Patentamt
European Patent Office
Office européen des brevets

(11) Veröffentlichungsnummer: **0 437 186 B1**

(12) # EUROPÄISCHE PATENTSCHRIFT

(45) Veröffentlichungstag der Patentschrift: **06.12.95**

(51) Int. Cl.6: **C08K 5/00**, C08L 57/08, C07D 251/70, //(C08K5/00, 5:3492,5:13),(C08K5/00,5:3492, 5:09)

(21) Anmeldenummer: **90811033.1**

(22) Anmeldetag: **27.12.90**

Die Akte enthält technische Angaben, die nach dem Eingang der Anmeldung eingereicht wurden und die nicht in dieser Patentschrift enthalten sind.

(54) **Melaminderivate als Stabilisatoren für chlorhaltige Polymerisate.**

(30) Priorität: **09.01.90 CH 56/90**

(43) Veröffentlichungstag der Anmeldung:
**17.07.91 Patentblatt 91/29**

(45) Bekanntmachung des Hinweises auf die Patenterteilung:
**06.12.95 Patentblatt 95/49**

(84) Benannte Vertragsstaaten:
**DE FR GB IT**

(56) Entgegenhaltungen:
**DE-A- 1 569 597**
**JP-A-54 006 043**
**US-A- 3 084 135**

**CHEMICAL ABSTRACTS, vol. 90, no. 24, 11 Juni 1979 Columbus, Ohio, USA & JP-A-79 006 043.**

**JOURNAL OF THE AMERICAN PHARMACEUTICAL ASSOCIATION. vol. 39, 1950, WASHINGTON US Seiten 393 - 396; D.F. WALKER ET AL.: "ARYLAMINOHETEROCYCLES. V. ANILINO-S-TRIAZINES"**

**Database CA (STN),Zusamm.Nr.90(24):187960m**

(73) Patentinhaber: **CIBA-GEIGY AG**
**Klybeckstrasse 141**
**CH-4002 Basel (CH)**

(72) Erfinder: **Wehner, Wolfgang, Dr.**
**Wetzbach 34**
**W-6144 Zwingenberg (DE)**
Erfinder: **Köstler, Hans-Günter**
**Gerhart-Hauptmannstrasse 2**
**W-6148 Heppenheim/Bergstrasse (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 437 186 B1

## Beschreibung

Die vorliegende Erfindung betrifft Zusammensetzungen enthaltend a) ein chlorhaltiges Polymerisat, b) ein Melaminderivat und c) ein Metallsalz, sowie die Verwendung eines Gemisches aus den Komponenten b) und c) zum Stabilisieren eines chlorhaltigen Polymerisats gegen thermischen Abbau.

Es ist bekannt, dass chlorhaltige Polymerisate gegen den schädigenden Einfluss von Licht und Wärme, insbesondere bei der Verarbeitung zu Formteilen, geschützt werden müssen. Einige Melaminderivate und deren Verwendung als Stabilisatoren für chlorhaltige Polymerisate werden beispielsweise in JP-A-Sho 56/43342, US-A-3 084 135 und US-A-3,496,136 beschrieben. Die Herstellung verschiedener Melaminderivate wurde bereits 1950 von D.F. Walker et al.; "J. Am. Pharm. Ass. 39, 393-396", 1951 von D.W. Kaiser et al.; "J. Am. Chem. Soc. 73, 2984-2986", 1963 von D.E. O'Brien et al.; "J. Med. Chem. 6, 467-471", 1967 von Smolin et al.; "s-Triazines and Derivatives - The Chemistry of Heterocyclic Compounds, Chapter VI, pp. 309-388" und ebenfalls 1967 von A.B. Borkovec et al.; "J. Med. Chem. 10, 457-461" sowie in US-A-4,312,988 beschrieben. Melaminderivate als geruchsbindende Mittel für Polyvinylchloridfilme sind aus den JP-A-79/6043 und JP-A-86/116512 bekannt.

Ein Gegenstand der vorliegenden Erfindung ist eine Zusammensetzung enthaltend

a) ein chlorhaltiges Polymerisat,

b) 0,01 bis 0,9 Gew.%, bezogen auf das chlorhaltige Polymerisat, einer Verbindung der Formel I und/oder deren Hydrochlorids,

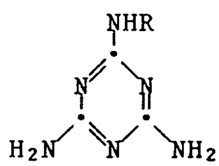

(I)

worin R $C_6$-$C_{20}$-Alkyl, durch 1 bis 5 Sauerstoffatome unterbrochenes $C_3$-$C_{20}$-Alkyl, durch 1 bis 5 OH substituiertes $C_1$-$C_{20}$-Alkyl, $C_3$-$C_8$-Cycloalkyl, durch $C_1$-$C_4$-Alkyl substituiertes $C_3$-$C_8$-Cycloalkyl, $C_7$-$C_{11}$-Phenylalkyl, am Phenyl durch 1 bis 3 Reste substituiertes $C_7$-$C_{11}$-Phenylalkyl, wobei die Reste unabhängig voneinander Hydroxy, Chlor, $C_1$-$C_4$-Alkyl, Methoxy oder Ethoxy bedeuten, oder R ferner eine Gruppe der Formel IIa oder IIb ist,

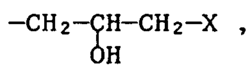 ,

(IIa)

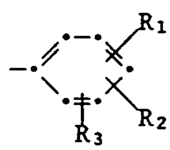

(IIb)

worin X $C_1$-$C_{20}$-Alkyloxy, $C_1$-$C_{20}$-Alkylthio, Phenyloxy, Phenylthio, Benzyloxy oder Benzylthio bedeutet und $R_1$, $R_2$ und $R_3$ unabhängig voneinander Wasserstoff, $C_1$-$C_{20}$-Alkyl, $C_1$-$C_{20}$-Alkyloxy, $C_1$-$C_{20}$-Alkylthio, $C_2$-$C_{12}$-Alkyloxycarbonyl, $C_2$-$C_{12}$-Alkanoyl, Phenyl, Phenyloxy, Phenylthio, Hydroxy, Mercapto oder Chlor sind, und

c) 0,01 bis 5 Gew.%, bezogen auf das chlorhaltige Polymerisat, eines Me(II)-Carboxylats und/oder Me(II)-Phenolats, wobei Me(II) Ba, Ca, Mg, Cd oder Zn bedeutet.

Die Komponente c) ist bevorzugt ein Gemisch aus Ba/Zn-Carboxylaten, wenn die Komponente b) Phenylmelamin ist.

R bedeutet als $C_6$-$C_{20}$-Alkyl z.B. Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl oder Icosyl.

R bedeutet als $C_3$-$C_{20}$-Alkyl, welches durch 1 bis 5 Sauerstoffatome unterbrochen ist, bevorzugt eine Gruppe der Formel

$$-\left[C_rH_{2r}-O\right]_s-Z \quad ,$$

worin r 2 oder 3 ist, s 1, 2, 3, 4 oder 5 bedeutet und Z z.B. Methyl, Ethyl, Propyl oder Butyl darstellt.

Beispiele für R als $C_1$-$C_{20}$-Alkyl, welches durch 1 bis 5 OH substituiert ist, sind Hydroxymethyl, 2-Hydroxyethyl, 3-Hydroxypropyl, 2,3-Dihydroxypropyl, 4-Hydroxybutyl, 5-Hydroxypentyl, 2-Hydroxypentyl, 2,3,4,5,6-Pentahydroxyhexyl, 8-Hydroxyoctyl, 2-Hydroxyoctyl, 2-Hydroxynonyl, 2-Hydroxydecyl oder 2-Hydroxyoctadecyl. $C_1$-$C_8$-Hydroxyalkyl, worin sich die Hydroxygruppe in Endstellung oder in 2-Stellung befindet, ist ebenso bevorzugt wie in 2-Stellung durch -OH substituiertes $C_9$-$C_{20}$-Alkyl. Durch -OH substituiertes $C_1$-$C_6$-Alkyl ist besonders bevorzugt.

Beispiele für R als $C_3$-$C_8$-Cycloalkyl, welches gegebenenfalls durch $C_1$-$C_4$-Alkyl substituiert sein kann, sind Cyclopropyl, Cyclobutyl, Cyclopentyl, Cyclohexyl, Methylcyclohexyl, 4-Butylcyclohexyl, Cycloheptyl, Cyclooctyl oder Cyclododecyl. $C_5$-$C_7$-Cycloalkyl, insbesondere Cyclohexyl, ist bevorzugt.

R bedeutet als $C_7$-$C_{11}$-Phenylalkyl z.B. Benzyl oder Phenylethyl.

Beispiele für R als $C_7$-$C_{11}$-Phenylalkyl, welches durch 1 bis 3 definitionsgemässe Reste substituiert ist, sind o-, m- oder p-Chlorbenzyl, 2,3-Dichlorbenzyl, 2,4-Dichlorbenzyl, 2,5-Dichlorbenzyl, 2,6-Dichlorbenzyl, 3,4-Dichlorbenzyl, 2,4,5-Trichlorbenzyl, 2,4,6-Trichlorbenzyl, o-, m- oder p-Hydroxybenzyl, o-, m- oder p-Methylbenzyl, 2,3-Dimethylbenzyl, 2,4-Dimethylbenzyl, 2,5-Dimethylbenzyl, 2,6-Dimethylbenzyl, 3,4-Dimethylbenzyl, 3,5-Dimethylbenzyl, 2-Methyl-4-tert-butylbenzyl, 2-Ethylbenzyl, 2,6-Diethylbenzyl, 2,6-Diethyl-4-methylbenzyl, 2,6-Diisopropylbenzyl, 4-tert-Butylbenzyl, 2-Chlor-6-methylbenzyl, 3-Chlor-2-methylbenzyl, 3-Chlor-4-methylbenzyl, 4-Chlor-2-methylbenzyl, 5-Chlor-2-methylbenzyl, 2,6-Dichlor-3-methylbenzyl, 2-Hydroxy-4-methylbenzyl, 3-Hydroxy-4-methylbenzyl, o-, m- oder p- Methoxybenzyl, o-, m- oder p-Ethoxybenzyl, 2,4-Dimethoxybenzyl, 2,5-Dimethoxybenzyl, 2,5-Diethoxybenzyl, 2-Methoxy-5-methylbenzyl, 4-Methoxy-2-methylbenzyl, 3-Chlor-4-methoxybenzyl, 3-Chlor-6-methoxybenzyl, 3-Chlor-4,6-dimethoxybenzyl oder 4-Chlor-2,5-dimethoxybenzyl.

Beispiele für $R_1$, $R_2$ und $R_3$ als $C_1$-$C_{20}$-Alkyl sind ausser den oben für R = Alkyl angegebenen Bedeutungen Methyl, Ethyl, Propyl, Isopropyl, Butyl, tert-Butyl, Pentyl, Hexyl und Heptyl.

X, $R_1$, $R_2$ und $R_3$ bedeuten als $C_1$-$C_{20}$-Alkyloxy z.B. Methoxy, Ethoxy, Butoxy, Pentyloxy, Hexyloxy, Heptyloxy, Octyloxy, Nonyloxy, Decyloxy, Dodecyloxy, Tridecyloxy, Hexadecyloxy oder Octadecyloxy. Eine der bevorzugten Bedeutungen für $R_1$, $R_2$ und $R_3$ sind $C_1$-$C_4$-Alkyloxy, insbesondere Methoxy und Ethoxy.

X, $R_1$, $R_2$ und $R_3$ bedeuten als $C_1$-$C_{20}$-Alkylthio z.B. Methylthio, Ethylthio, Propylthio, Butylthio, Pentylthio, Hexylthio, Heptylthio, Octylthio, Nonylthio, Decylthio, Dodecylthio, Tridecylthio, Hexadecylthio oder Octadecylthio. $C_8$-$C_{18}$-Alkylthio ist bevorzugt.

Beispiele für $R_1$, $R_2$ und $R_3$ als $C_2$-$C_{12}$-Alkyloxycarbonyl sind Methoxycarbonyl, Ethoxycarbonyl, Propoxycarbonyl, Butoxycarbonyl, Pentyloxycarbonyl, Hexyloxycarbonyl, Octyloxycarbonyl und Decyloxycarbonyl.

$R_1$, $R_2$ und $R_3$ bedeuten als $C_2$-$C_{12}$-Alkanoyl z.B. Acetyl, Propanoyl, Butanoyl, Pentanoyl, Hexanoyl, Heptanoyl, Octanoyl oder Decanoyl.

Beispiele für die Gruppe IIb sind o-, m- oder p-Chlorphenyl, 2,3-Dichlorphenyl, 2,4-Dichlorphenyl, 2,5-Dichlorphenyl, 3,4-Dichlorphenyl, 2,4,5-Trichlorphenyl, 2,4,6-Trichlorphenyl, o-, m- oder p-Hydroxyphenyl, o-, m- oder p-Methylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2-Methyl-4-tert-butylphenyl, 2-Ethylphenyl, 2,3-Diethylphenyl, 2,4-Diethylphenyl, 2,5-Diethylphenyl, 2,6-Diethylphenyl, 3,5-Diethylphenyl, 2,6-Diethyl-4-methylphenyl, 2,6-Diisopropylphenyl, 4-tert-Butylphenyl, 3,5-Di-tert-butylphenyl, 2-Chlor-6-methylphenyl, 3-Chlor-2-methylphenyl, 3-Chlor-4-methylphenyl, 4-Chlor-2-methylphenyl, 5-Chlor-2-methylphenyl, 2,6-Dichlor-3-methylphenyl, o-, m- oder p-Methoxyphenyl, o-, m- oder p-Ethoxyphenyl, o-, m- oder p-Propoxyphenyl, o-, m- oder p-Butoxyphenyl, o-, m- oder p-Hexyloxyphenyl, o-, m- oder p-Octyloxyphenyl, 2,4-Dimethoxyphenyl, 2,5-Dimethoxyphenyl, 2,5-Diethoxyphenyl, 2-Methoxy-5-methylphenyl, 4-Methoxy-2-methylphenyl, 3-Chlor-4-methoxyphenyl, 3-Chlor-6-methoxyphenyl, 3-Chlor-4,6-dimethoxyphenyl, 4-Chlor-2,5-dimethoxyphenyl o-, m- oder p-Methylthiophenyl, o-, m- oder p-Ethylthiophenyl, o-, m- oder p-Propylthiophenyl, o-, m- oder p-Butylthiophenyl, o-, m- oder p-Pentylthiophenyl, o-, m- oder p-Hexylthiophenyl, o-, m- oder p-Heptylthiophenyl, o-, m- oder p-Octylthiophenyl, o-, m- oder p-Nonylthiophenyl, o-, m- oder p-Decylthiophenyl o-, m- oder p-Phenylphenyl und 2-Hydroxy-4-methylphenyl, 3-Hydroxy-4-methylphenyl sowie eine Gruppe der Formel

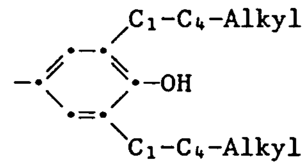 .

Bevorzugte Bedeutungen für die Gruppe IIb sind den weiter unten angegebenen Beispielen 1 bis 8 zu entnehmen.

In der Gruppe der Formel IIb bedeuten $R_1$ und/oder $R_2$ besonders bevorzugt Wasserstoff.

Von Interesse sind Zusammensetzungen, worin R $C_{10}$-$C_{18}$-Alkyl, durch OH substituiertes $C_1$-$C_{10}$-Alkyl, $C_5$-$C_7$-Cycloalkyl, durch $C_1$-$C_4$-Alkyl substituiertes $C_5$-$C_7$-Cycloalkyl, Benzyl, am Phenyl durch 1 bis 3 Reste substituiertes Benzyl, wobei die Reste unabhängig voneinander Hydroxy, Chlor, $C_1$-$C_4$-Alkyl, Methoxy oder Ethoxy bedeuten, oder R ferner eine Gruppe der Formel IIa oder IIb ist, worin X $C_1$-$C_{10}$-Alkyloxy, $C_8$-$C_{18}$-Alkylthio, Phenyloxy, Phenylthio, Benzyloxy oder Benzylthio bedeutet und $R_1$, $R_2$ und $R_3$ unabhängig voneinander Wasserstoff, $C_1$-$C_{10}$-Alkyl, $C_1$-$C_{10}$-Alkyloxy, $C_8$-$C_{18}$-Alkylthio, $C_2$-$C_{12}$-Alkyloxycarbonyl, $C_2$-$C_6$-Alkanoyl, Phenyloxy, Phenylthio, Hydroxy, Mercapto oder Chlor sind.

Ebenfalls von Interesse sind Zusammensetzungen, worin R $C_{10}$-$C_{18}$-Alkyl, durch OH substituiertes $C_1$-$C_6$-Alkyl, Cyclohexyl oder Benzyl bedeutet.

R bedeutet bevorzugt die Gruppe IIb.

Ebenfalls bevorzugt sind Zusammensetzungen, worin R eine Gruppe der Formel IIb ist und die Reste $R_1$, $R_2$ und $R_3$ unabhängig voneinander Wasserstoff, $C_1$-$C_4$-Alkyl, $C_1$-$C_4$-Alkyloxy, $C_8$-$C_{16}$-Alkylthio, $C_2$-$C_{12}$-Alkyloxycarbonyl, Hydroxy, Mercapto oder Chlor bedeuten.

Besonders bevorzugt sind Zusammensetzungen, worin R eine Gruppe der Formel IIb ist, $R_1$ Wasserstoff bedeutet, $R_2$ und $R_3$ unabhängig voneinander $C_1$-$C_4$-Alkyl, $C_1$-$C_4$-Alkyloxy, $C_8$-$C_{16}$-Alkylthio, $C_2$-$C_{12}$-Alkyloxycarbonyl, Hydroxy oder Chlor bedeuten und $R_2$ zusätzlich Wasserstoff ist.

Von besonderem Interesse sind Zusammensetzungen, worin R eine Gruppe der Formel IIb darstellt, $R_1$ und $R_2$ Wasserstoff sind und $R_3$ $C_1$-$C_4$-Alkyl, $C_1$-$C_4$-Alkyloxy, $C_8$-$C_{16}$-Alkylthio, $C_2$-$C_{12}$-Alkyloxycarbonyl, Hydroxy oder Chlor, insbesondere $C_1$-$C_4$-Alkyloxy oder Hydroxy, bedeutet.

Gemäss einer weiteren Bevorzugung kann die Komponente b) als Hydrochlorid vorliegen.

Die Komponente c) ist bevorzugt ein Me(II)-Carboxylat, wobei Me(II) Ba, Ca, Mg, Cd oder Zn bedeutet. Bei den Carboxylaten handelt es sich bevorzugt um Salze von Carbonsäuren mit 7 bis 20 C-Atomen, z.B. Benzoate, Alkanoate oder Alkenoate, bevorzugt Stearate, Oleate, Laurate, Palmitate, Hydroxystearate oder 2-Ethylhexanoate. Besonders bevorzugt sind Stearate, Oleate oder p-tert-Butylbenzoate.

Gemische aus Ba/Zn- oder Ca/Zn-Carboxylaten werden als Komponente c) ebenfalls besonders bevorzugt.

Bedeutet die Komponente c) ein Me(II)-Phenolat, so handelt es sich insbesondere um $C_7$-$C_{20}$-(o-, m- oder p-)Alkylphenolate, beispielsweise o-, m- oder p-Nonylphenolat.

Gemäss einer weiteren Bevorzugung enthalten die erfindungsgemässen Zusammensetzungen als zusätzliche Komponente d) eine Epoxyverbindung und/oder ein Phosphit.

Bei der Epoxyverbindung handelt es sich bevorzugt um epoxidierte Oele und epoxidierte Fettsäureester, z.B. epoxidiertes Sojabohnenöl, epoxidiertes Butyloleat und epoxidiertes Octyloleat.

Bei den Phosphiten handelt es sich bevorzugt um solche der Formeln

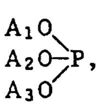, 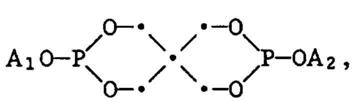, 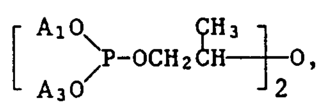, 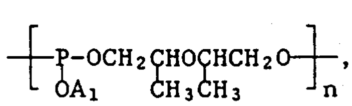,

worin $A_1$, $A_2$ und $A_3$ unabhängig voneinander $C_4$-$C_{18}$-Alkyl, $C_6$-$C_{18}$-Alkenyl, $C_5$-$C_7$-Cycloalkyl, Phenyl oder durch ein bis drei $C_1$-$C_{12}$-Alkylgruppen substituiertes Phenyl bedeuten.

Beispiele sind Trioctyl-, Tridecyl-, Tridodecyl-, Tritetradecyl-, Tristearyl-, Trioleyl-, Triphenyl-, Trikresyl-, Tris-p-nonylphenyl- und Tricyclohexylphosphit. Bevorzugt sind die Aryldialkyl- sowie die Alkyldiarylphosphite, wie z.B. Phenyldidecyl-, (2,4-Di-tert-butylphenyl)didodecyl-, (2,6-Di-tert-butylphenyl)didodecylphosphit und die Dialkyl- und Diaryl-pentaerythrit-diphosphite, wie z.B. Distearylpentaerythrit-diphosphit. Ebenfalls bevorzugt sind die Tetraphenyl- und Tetraalkyl-[dipropylenglykol-1,2]-diphosphite und die Poly-[dipropylenglykol-1,2-phenylphosphite] sowie die Poly-[dipropylenglykol-1,2-alkylphosphite].

Besonders bevorzugte organische Phosphite sind Distearyl-pentaerythritdiphosphit, Tris(nonylphenyl)-phosphit, Phenyldidecylphosphit, Tetraphenyl-[dipropylenglykol-1,2]-diphosphit und Poly-[dipropylenglykol-1,2-phenylphosphit].

Die Me(II)-Carboxylate oder -Phenolate werden bevorzugt in Mengen von 0,05 bis 5 Gew.% eingesetzt.

Die Phosphite werden z.B. in Konzentrationen von 0,3 bis 5, vorzugsweise 0,5 bis 1 Gew.% und die Epoxyverbindungen, wie z.B. das epoxidierte Sojabohnenöl, zweckmässig in Konzentrationen von 1 bis 8, vorzugsweise 1 bis 3 Gew.% eingesetzt.

Die Verbindungen der Formel I werden bevorzugt in Mengen von 0,05 bis 0,9 oder insbesondere 0,1 bis 0,7 Gew.% in das chlorhaltige Polymerisat eingearbeitet.

Die Angabe Gew.% bezieht sich jeweils auf das zu stabilisierende Material.

Bei den chlorhaltigen Polymerisaten handelt es sich bevorzugt um Vinylchloridhomopolymere oder -copolymere. Als Comonomere für die Copolymerisate kommen z.B. in Frage: Vinylacetat, Vinylidenchlorid, Transdichlorethen, Ethylen, Propylen, Butylen, Maleinsäure, Acrylsäure, Fumarsäure, Itaconsäure. Weitere geeignete chlorhaltige Polymere sind nachchloriertes PVC und chlorierte Polyolefine, ferner Pfropfpolymerisate von PVC mit EVA, ABS und MBS. Bevorzugte Substrate sind auch Mischungen der vorstehend genannten Homo- und Copolymerisate, insbesondere Vinylchlorid-Homopolymerisate, mit anderen thermoplastischen oder/und elastomeren Polymeren, insbesondere mit ABS, MBS, NBR, SAN, EVA.

Weiterhin bevorzugt sind Suspensions- und Massepolymere sowie Emulsionspolymere.

Als chlorhaltiges Polymerisat ist Polyvinylchlorid besonders bevorzugt.

Ein weiterer Gegenstand der Erfindung ist die Verwendung eines Stabilisatorsystems enthaltend die Komponenten b) und c) zum Stabilisieren eines chlorhaltigen Polymerisats gegen thermischen Abbau.

Je nach dem Verwendungszweck der Polymerisate können vor oder bei der Einarbeitung der Stabilisatoren auch weitere Zusätze eingearbeitet werden, wie zum Beispiel phenolische Antioxidantien, Gleitmittel (bevorzugt Montanwachse oder Glycerinester, Fettsäureester, Paraffine, Amidwachse, Stearinsäure, Mono- und Dihydroxystearinsäure, höhere Fettalkohole), Weichmacher, Füllstoffe, Russ, Asbest, Kaolin, Talk, Glasfasern, Modifikatoren (wie etwa Schlagzäh-Zusätze), Verarbeitungshilfen (z.B. Polymethacrylsäureester), optische Aufheller, Pigmente, Lichtschutzmittel, sterisch gehinderte Amine, UV-Absorber, Flammschutzmittel oder Antistatika.

Weitere mögliche Zusätze sind ferner β-Aminocrotonate, z.B. die in DE-A-804 442, DE-A-807 207 und JP-A-75/17454 beschriebenen Verbindungen, Pyrrole, z.B. die in EP-A-22 087 angegebenen Verbindungen, Aminouracile, z.B. die in EP-A-65 934 offenbarten Verbindungen, Aminothiouracile, z.B. die aus EP-A-41 479 bekannten Verbindungen, Polyole, z.B. die in DE-A-3 019 910 beschriebenen Verbindungen, β-Diketone, z.B. die in DE-A-2 600 516 angegebenen Verbindungen, Hydrotalcite, insbesondere die in der DE-A-3 843 581 beschriebenen Verbindungen, oder auch Gemische aus β-Diketonen und Hydrotalciten, wie z.B. in EP-A-63 180 beschrieben, ferner Mg-Al-Carbonate, z.B. die in JP-A-Sho 62/267347 (= Chemical Abstracts 108:168 635 h) offenbarten Mg-Al-Carbonate, sowie Alcamizer, z.B. 4 MgO $Al_2O_3 \bullet CO_2 \bullet 9\ H_2O$, 4 MgO $Al_2O_3 \bullet CO_2 \bullet 6\ H_2O$, ZnO 3MgO $Al_2O_3 \bullet CO_2 \bullet$8-9 $H_2O$, ZnO 3 MgO $Al_2O_3 \bullet CO_2 \bullet$5-6 $H_2O$ und $Mg_4Al_2(OH)_{12}(CO_3)_{1-x/2}(ClO_4)_x mH_2O$.

Als mögliche Zusätze sind auch chemische Treibmittel für geschäumtes Hart- oder Weich-Polyvinylchlorid wie z.B. $NaHCO_3$ und die in "Gächter/Müller; Taschenbuch der Kunststoff-Additive, Carl Hanser Verlag München-Wien, 2. Ausgabe, 1983" auf der Seite 651 angegebenen Treibmittel zu nennen.

Die Einarbeitung der Stabilisatorkomponenten in das chlorhaltige Polymerisat erfolgt am günstigsten, wie üblich, auf einem Mischwalzwerk, z.B. einem 2-Walzenstuhl bei Temperaturen zwischen 150° und 200°C. Im allgemeinen lässt sich eine genügende Homogenisierung innerhalb von 5 bis 15 Minuten erreichen. Die Zugabe der Komponenten kann einzeln oder gemeinsam als Vorgemisch erfolgen. Als zweckmässig hat sich ein flüssiges Vorgemisch erwiesen, d.h. es wird in Gegenwart von indifferenten Lösungsmitteln und/oder Weichmachern gearbeitet.

Die erfindungsgemässen Zusammensetzungen können nach den dafür gebräuchlichen Formgebungsverfahren, z.B. durch Extrusion, Spritzgiessen oder Kalandrieren zu Formteilen verarbeitet werden. Auch die Verwendung als Plastisole ist möglich.

Bevorzugt werden die erfindungsgemässen Zusammensetzungen für die Herstellung von Elektrokabeln, Hohlkörpern, z.B. Rohren, und insbesondere Folien in der Kraftfahrzeugindustrie verwendet. Diese Verwendung ist ebenfalls Gegenstand der Erfindung. Ein besonders bevorzugtes Einsatzgebiet ist die Herstellung von Folien für Kraftfahrzeuginnenräume, insbesondere wie sie in der DE-A 3 227 107 und der DE-A 3 401 482 beschrieben sind.

Die erfindungsgemässen Zusammensetzungen werden besonders vorteilhaft zur Herstellung von Tiefzieh- und Weichfolien auf PVC-Basis herangezogen, vor allem zur Verwendung in der Kraftfahrzeugindustrie.

Die Verbindungen der Formel I können in Analogie zu bekannten Verfahren hergestellt werden. Herstellungsverfahren sind z.B. in folgenden Veröffentlichungen beschrieben: D.F. Walker et al.; J. Am. Pharm. Assoc. 39, 393 (1950), D.W. Kaiser et al.; J. Am. Chem. Soc. 73, 2984 (1951), A.B. Borkovec et al.; J. Med. Chem. 10, 457 (1967).

Wenn R eine der oben angegebenen Bedeutungen ausser der Gruppe IIb annimmt, ist es zweckmässig, die Melaminderivate nach folgendem Schema herzustellen:

Schema A

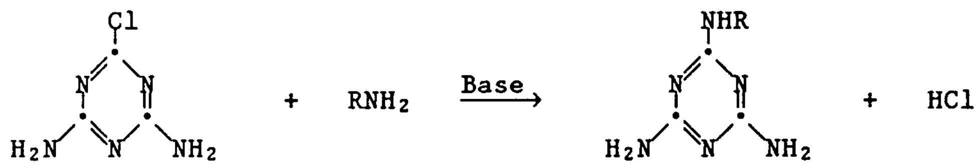


Im allgemeinen ist Wasser ein geeignetes Reaktionsmedium. In einigen Fällen, insbesondere wenn R einen langkettigen Alkylrest bedeutet, ist es vorteilhaft, einen Lösevermittler, z.B. Dimethylacetamid, zuzusetzen. Bei der Base handelt es sich z.B. um Alkalihydroxid, bevorzugt Natriumhydroxid.

Das erhaltene Melamin kann durch Erwärmen mit konzentrierter Salzsäure (geringer Ueberschuss) in das entsprechende Hydrochlorid übergeführt werden. Umgekehrt ist es möglich, aus dem Hydrochlorid durch Behandlung mit z.B. Alkalihydroxid- oder Alkalibicarbonat-Lösung wiederum das Melamin zu erhalten, was einen weiteren Reinigungsschritt darstellen kann.

In einigen Fällen ist es auch möglich, Verbindungen der Formel I, worin R eine Gruppe der Formel IIb ist, nach dem obigen Schema A herzustellen; allerdings muss in den Fällen eine erhöhte Aminbasizität vorliegen.

Bevorzugt werden die Verbindungen der Formel I, worin R eine Gruppe der Formel IIb ist, nach dem unten angegebenen Schema B hergestellt.

Schema B:

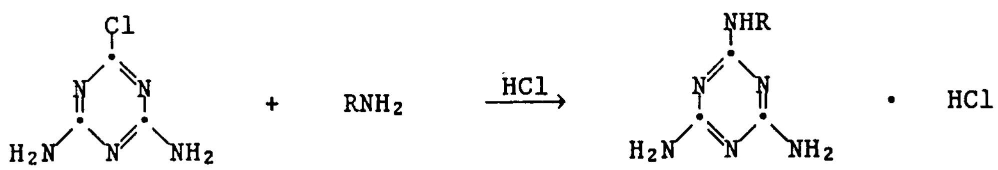


Auch in diesem Fall ist Wasser ein geeignetes Reaktionsmedium, wobei, falls erforderlich, wiederum ein Lösevermittler zugegeben werden kann. Die Ueberführung des Hydrochlorids in das entsprechende Melaminderivat erfolgt zweckmässigerweise wie oben beschrieben.

Im allgemeinen weisen die Verbindungen der Formel I sowie deren Hydrochloride einen definierten Hydratwassergehalt auf.

Die Ausgangsprodukte sind im Handel erhältlich oder können in Analogie zu dem Fachmann bekannten Verfahren hergestellt werden. Die Herstellung von Diamino-chlortriazin wird z.B. von J.T. Thurston et al. in "J. Am. Chem. Soc. 73, 2981 (1951)" beschrieben.

Die Verbindungen der Formel I können auch gegen den photolytischen Abbau von chlorhaltigen Polymerisaten eingesetzt werden.

Die folgenden Beispiele erläutern die Erfindung weiter. Teile- und Prozentangaben beziehen sich darin, soweit nichts anderes angegeben ist, auf das Gewicht.

Beispiel 1: Herstellung von N-(n-Dodecyl)melamin (Verbindung 1-A-1).

Ein Gemisch aus 14,6 g (0,1 Mol) Diamino-chlortriazin, 19,5 g (0,105 Mol) n-Dodecylamin und 4,0 g (0,1 Mol) Aetznatron in 200 ml Wasser wird 5 Stunden am Rückfluss erhitzt. Der gebildete Feststoff wird

abgesaugt, chloridfrei gewaschen und anschliessend mit einem Gemisch aus Methylenchlorid/Ether (1:1) behandelt. Der Niederschlag wird abfiltriert und mit Dimethylformamid ausgekocht. Nach Filtration wird das aufkonzentrierte Filtrat in Wasser eingerührt. Der entstandene Niederschlag wird bis zur Gewichtskonstanz getrocknet. Die Ausbeute beträgt 65 % der Theorie. Das Produkt besitzt 0,1 Mol Hydratwasser pro Mol Melamin und schmilzt bei 119 ° C.

Beispiel 2:

Die in Tabelle 1 angegebenen Verbindungen werden in Analogie zu dem in Beispiel 1 beschriebenen Verfahren hergestellt.

Tabelle 1:

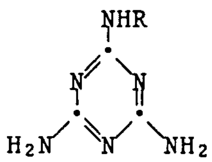

| Verbindung Nr. | R | Schmelzpunkt | Ausbeute [% der Theorie] |
|---|---|---|---|
| 1-A-2 | $-(CH_2)_3-OH$ | 160°C | 87 |
| 1-A-3 | $-(CH_2)_5-OH$ | - | - |
| 1-A-4 | $-CH_2-CH=CH_2$ | 172°C | 57,2 (Produkt liegt als Hydrat vor (0,25 Mol $H_2O$ pro Mol Melamin).) |
| 1-A-5 | 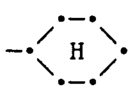 | 146°C | 66 (Nach Umkristallisation aus Essigsäure. Produkt liegt als Hydrat vor (0,6 Mol $H_2O$ pro Mol Melamin).) |
| 1-A-6 | $-CH_2-$ 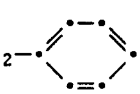 | | |

Beispiel 3: Herstellung von N-Benzylmelaminhydrochlorid (Verbindung 1-A-7).

10,8 g (0,05 Mol) der Verbindung 1-A-6 werden mit 6,84 g (0,06 Mol) 32 %iger Salzsäure unter Rühren 1 Stunde am Rückfluss erhitzt. Die klare Lösung wird auf 0 ° C abgekühlt, die gebildeten Kristalle filtriert, gewaschen und getrocknet. Die Ausbeute beträgt 73,8 % der Theorie. Das Produkt besitzt einen Schmelzpunkt von 275 ° C.

Beispiel 3a: Herstellung von N-[3-(2'-Ethylhexyl)oxy-2-hydroxypropyl]-melamin (Verbindung 1-A-8).

Ein Gemisch aus 29,1 g (0,2 Mol) Diamino-chlortriazin, 40,7 g (0,2 Mol) 3-(2'-Ethylhexyl)oxy-2-hydroxypropylamin und 300 ml Wasser wird am Rückfluss erhitzt. Innerhalb von 1 Stunde werden 0,2 Mol NaOH in 80 ml Wasser zugetropft. Anschliessend wird das Reaktionsgemisch 2 Stunden am Rückfluss erhitzt. Zu dem erhaltenen Oel werden 25 ml konzentrierte Salzsäure gegeben. Es liegt eine klare Lösung

vor. Beim Abkühlen fällt das Hydrochlorid von N-[3-(2'-Ethylhexyl)oxy-2-hydroxypropyl]melamin aus, welches mit $NaHCO_3$ bei 40 °C neutralisiert wird. Das erhaltene wachsartige Produkt wird mit Eiswasser chloridfrei gewaschen und im Vakuum getrocknet. Die Ausbeute beträgt 86,6 % der Theorie. Das Produkt besitzt einen Schmelzpunkt von 109 - 115 °C.

Beispiel 3b: Herstellung von N-n-Hexylmelamin (Verbindung 1-A-9).

Die Herstellung erfolgt in Analogie zu Beispiel 3a. Die Ausbeute beträgt 70,8 % der Theorie. Das Produkt besitzt einen Schmelzpunkt von 116-119 °C.

Beispiel 4: Herstellung von N-Phenylmelamin (= Verbindung 2-A-1).

Ein Gemisch aus 14,6 g (0,1 Mol) Diamino-chlortriazin, 9,8 g (0,105 Mol) Anilin und 4,0 g (0,1 Mol) Aetznatron in 30 ml Wasser wird unter Zusatz von 200 ml Wasser 2 Stunden am Rückfluss erhitzt. Die trübe Lösung wird abgekühlt, der ausgefallene Feststoff filtriert, chloridfrei gewaschen und bis zur Gewichtskonstanz getrocknet. Die Reinigung des erhaltenen Produkts erfolgt durch Lösen in Eisessig und anschliessende Bicarbonatbehandlung. Die Ausbeute beträgt 62 % der Theorie. Das Produkt besitzt einen Schmelzpunkt von 202 °C.

Beispiel 5:

Die in den Tabellen 2a bis 2c angegebenen Verbindungen werden in Analogie zu dem in Beispiel 4 beschriebenen Verfahren hergestellt. Die Angaben in den Tabellen 2a bis 2c beziehen sich auf die unten angegebene allgemeine Formel.

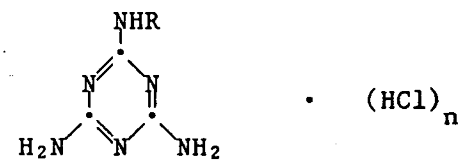

Tabelle 2a:

| Verb. Nr. | n | R | Schmelzpunkt | Ausbeute [% d.Theorie] |
|---|---|---|---|---|
| 2a-A-1 | 0 | 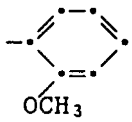 | 163°C | 98 (Produkt liegt als Hemihydrat vor.) |
| 2a-A-2 | 0 | 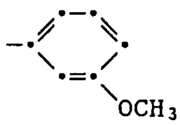 | 184°C | 96 |
| 2a-A-3 | 0 | 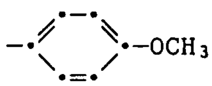 | 205°C | 95 |
| 2a-A-4 | 0 | 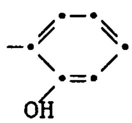 | 260°C | 76 (Nach Umkristallisation aus Ethanol.) |
| 2a-A-5 | 1 | 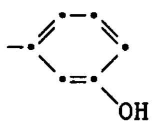 | 271°C | 36 (Produkt liegt als Hydrat vor (1,3 Mol $H_2O$ pro Mol Melamin).) |
| 2a-A-6 | 0 | 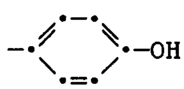 | 288°C | 39 (Nach Umkristallisation aus Methanol.) |

Tabelle 2b:

| Verb. Nr. | n | R | Schmelzpunkt | Ausbeute [% d.Theorie] |
|---|---|---|---|---|
| 2b-A-1 | 0 | 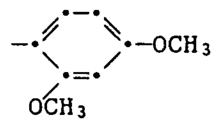 | 215°C | 97 |

Tabelle 2c:

| Verb. Nr. | n | R | Schmelzpunkt | Ausbeute [% d.Theorie] |
|---|---|---|---|---|
| 2c-A-1 | 0 | 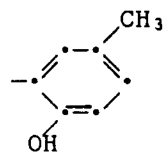 | 200°C | 70 (Nach Umkristallisation aus Ethanol/Wasser. Produkt liegt als Hemihydrat vor.) |
| 2c-A-2 | 0 | 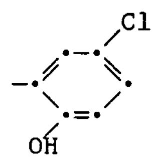 | 240°C | 58 (Nach Umkristallisation aus Ethanol/Wasser. Produkt liegt als Hydrat vor (0,75 Mol $H_2O$ pro Mol Melamin).) |

Beispiel 6: Herstellung von N-Phenylmelamin (Verbindung 1-B-1 (=2-A-1)).

Ein Gemisch aus 19,7 g (0,135 Mol) Diamino-chlortriazin, 15,5 g (0,140 Mol) Anilin, 1,3 ml konz. Salzsäure (Katalysator) wird in 200 ml Wasser unter Rühren 1 Stunde am Rückfluss erhitzt. Die Lösung wird filtriert und mit konz. Natriumhydrogencarbonatlösung neutralisiert (pH = 7,2). Der entstandene Niederschlag wird abgesaugt, chloridfrei gewaschen und bis zur Gewichtskonstanz getrocknet. Das erhaltene Produkt besitzt einen Schmelzpunkt von 206 ° C. Die Ausbeute beträgt 86,4 % der Theorie.

Beispiel 7: Herstellung von N-Phenylmelaminhemihydrochlorid (Verbindung 1-B-2).

Ein Gemisch aus 36,4 g (0,25 Mol) Diamino-chlortriazin, 24,4 g (0,26 Mol) Anilin und 7 ml konzentrierter Salzsäure wird in 500 ml Wasser 1 Stunde am Rückfluss erhitzt. Die Lösung wird filtriert und abgekühlt. Die erhaltenen farblosen Kristalle werden gewaschen und bis zur Gewichtskonstanz getrocknet. Die Ausbeute beträgt 68,3 % der Theorie. Das Produkt besitzt einen Schmelzpunkt von 259 ° C und liegt als Dihydrat vor.

Beispiel 8:

Die in den Tabellen 3a bis 3d angegebenen Verbindungen werden in Analogie zu dem in Beispiel 6 oder 7 beschriebenen Verfahren hergestellt. Die Angaben in den Tabellen 3a bis 3d beziehen sich auf die unten angegebene allgemeine Formel.

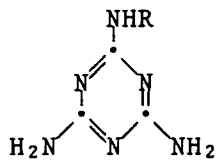 • $(HCl)_n$

Tabelle 3a:

| Verb. Nr. | n | R | Schmelzpunkt | Ausbeute [% d.Theorie] |
|---|---|---|---|---|
| 3a-B-1 | 0 | 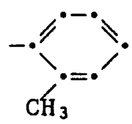 | 210°C | ~ 65 (Produkt liegt als Hydrat vor (0,1 Mol $H_2O$ pro Mol Melamin).) |
| 3a-B-2 | 0 | 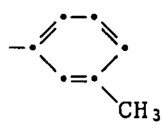 | 220°C | 88,2 (Produkt liegt als Hydrat vor (0,2 Mol $H_2O$ pro Mol Melamin).) |
| 3a-B-3 | 0 | 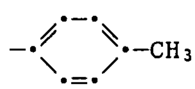 | 290°C | 83,8 (Produkt liegt als Hydrat vor (0,15 Mol $H_2O$ pro Mol Melamin).) |
| 3a-B-4 | 0 | 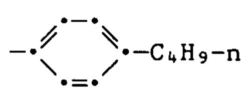 | 161°C | 43 (Nach Umkristallisation aus Methanol.) |
| 3a-B-5 | 0 | 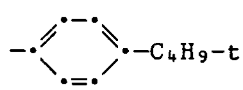 | 250°C | 76 |
| 3a-B-6 | 0 | 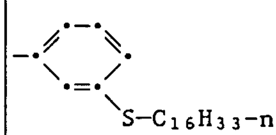 | 80°C | 81 (Dimethylacetamid-Zusatz. Produkt liegt als Sesquihydrat vor.) |
| 3a-B-7 | 0 | 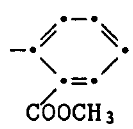 | 320°C (Zersetzung) | 75 (Bei der Umsetzung fällt auch die freie Säure an, die mit üblichen Methoden abgetrennt werden kann. Produkt liegt als Hydrat vor (0,2 Mol $H_2O$ pro Mol Melamin).) |

Fortsetzung Tabelle 3a:

| Verb. Nr. | n | R | Schmelzpunkt | Ausbeute [% d.Theorie] |
|---|---|---|---|---|
| 3a-B-8 | 0 | 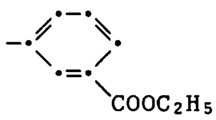 | 165°C | 58 (Nach Umkristallisation aus Essigsäure. Produkt liegt als Hydrat vor (0,2 Mol $H_2O$ pro Mol Melamin).) |
| 3a-B-9 | 0 | 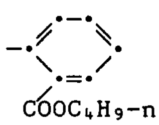 | 190°C | 28 (Dimethylacetamid-Zusatz. Produkt liegt als Hydrat vor (0,1 Mol $H_2O$ pro Mol Melamin).) |
| 3a-B-10 | 0 | 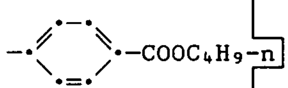 | 280°C | 77 |
| 3a-B-11 | 0 | 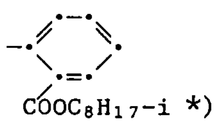 | 152°C | 64 (Herstellung durch Umesterung von 3a-B-7 in Gegenwart von $Ti(OC_4H_9)_4$.) |
| 3a-B-12 | 0 | 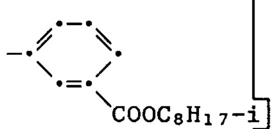 | 65°C | 36,5 (Herstellung durch Umesterung von 3a-B-8 in Gegenwart von $Ti(OC_4H_9)_4$. Reinigung durch Umkristallisation aus Methylenchlorid/Petrolether.) |
| 3a-B-13 | 0 | 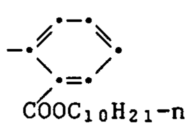 | 160°C | 43,8 (Dimethylacetamid-Zusatz.) |
| 3a-B-14 | 1 | 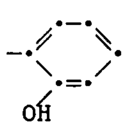 | 294°C | 73,1 (Nach Umkristallisation aus Wasser mit Aktivkohle. Produkt liegt als Hydrat vor (0,8 Mol $H_2O$ pro Mol Melamin).) |

*) $-C_8H_{17}$-i ≙ 2-Ethylhexyl

Fortsetzung Tabelle 3a:

| Verb. Nr. | n | R | Schmelzpunkt | Ausbeute [% d.Theorie] |
|---|---|---|---|---|
| 3a-B-15 | 0 | 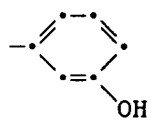 | 242°C | 89,9 (Herstellung aus dem Hydrochlorid mit Bicarbonat-Lösung.) |
| 3a-B-16 | 1 | 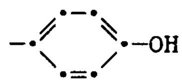 | 290°C | 73,8 (Nach Umkristallisation aus Wasser mit Aktivkohle. Produkt liegt als Monohydrat vor.) |
| 3a-B-17 | 0 | 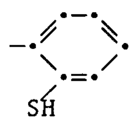 | 278°C | 49,3 (Dimethylacetamid-Zusatz. Produkt liegt als Hemihydrat vor.) |
| 3a-B-18 | 0 | 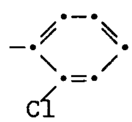 | 203°C | 89,8 |
| 3a-B-19 | 0 | 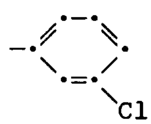 | 165°C | 85,0 |
| 3a-B-20 | 0 | 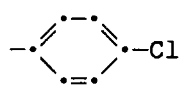 | 238°C | 87,9 |

Tabelle 3b:

| Verb. Nr. | n | R | Schmelzpunkt | Ausbeute [% d.Theorie] |
|---|---|---|---|---|
| 3b-B-1 | 0 | 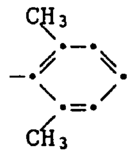 | 260°C | 78,2 (Produkt liegt als Hydrat vor (1,2 Mol $H_2O$ pro Mol Melamin).) |
| 3b-B-2 | 0 | 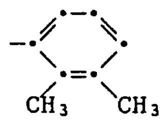 | 251°C | 76,8 (Produkt liegt als Hydrat vor (0,15 Mol $H_2O$ pro Mol Melamin).) |
| 3b-B-3 | 0 | 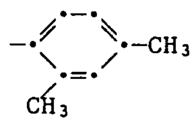 | 233°C | 87,0 (Produkt liegt als Hydrat vor (0,15 Mol $H_2O$ pro Mol Melamin).) |
| 3b-B-4 | 0 | 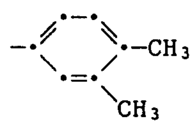 | 260°C | 88,8 (Produkt liegt als Hydrat vor (0,1 Mol $H_2O$ pro Mol Melamin).) |
| 3b-B-5 | 0 | 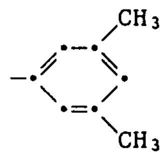 | 277°C | ~ 80 (Produkt liegt als Hydrat vor (0,15 Mol $H_2O$ pro Mol Melamin).) |
| 3b-B-6 | 0 | 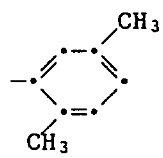 | 230°C | 88,5 (Produkt liegt als Hydrat vor (0,1 Mol $H_2O$ pro Mol Melamin).) |
| 3b-B-7 | 1 | 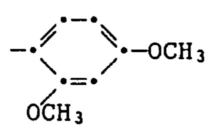 | 265°C | 61,5 (Nach Umkristallisation aus Wasser m. Aktivkohle. Das Produkt liegt als Monohydrat vor.) |
| 3b-B-8 | 1 | 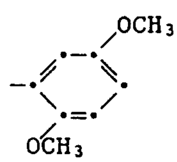 | 256°C | 76,0 (Nach Umkristallisation aus Wasser mit Aktivkohle. Produkt liegt als Hydrat vor (0,2 Mol $H_2O$ pro Mol Melamin).) |

Fortsetzung Tabelle 3b:

| Verb. Nr. | n | R | Schmelzpunkt | Ausbeute [% d.Theorie] |
|---|---|---|---|---|
| 3b-B-9 | 0 | 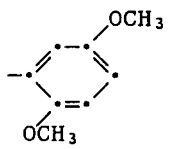 | 186°C | 99,0 (Hergestellt durch Behandeln von 3b-B-8 mit Bicarbonat-Lösung.) |
| 3b-B-10 | 1 | 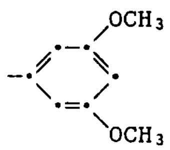 | 277°C | 71,4 (Nach Umkristallisation aus Wasser mit Aktivkohle.) |
| 3b-B-11 | 0 | 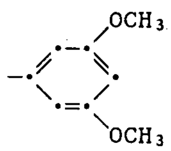 | 103°C | 87,0 (Hergestellt durch Behandeln von 3b-B-10 mit Bicarbonat-Lösung. Produkt liegt als Monohydrat vor.) |
| 3b-B-12 | 1 | 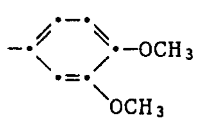 | - | 79,5 (Nach Umkristallisation aus Wasser mit Aktivkohle. Produkt liegt als Hydrat vor (0,2 Mol $H_2O$ pro Mol Melamin).) |
| 3b-B-13 | 0 | 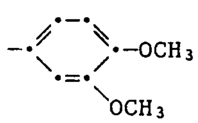 | 225°C | 99,4 (Hergestellt durch Behandeln von 3b-B-12 mit Bicarbonat-Lösung. Produkt liegt als Hydrat vor (0,2 Mol $H_2O$ pro Mol Melamin).) |
| 3b-B-14 | 0 | 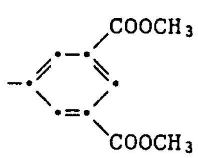 | 267°C | 50 (Produkt liegt als Hemihydrat vor.) |
| 3b-B-15 | 0 | 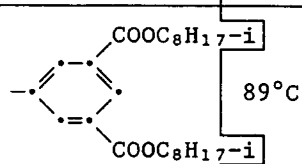 | 89°C | 51 (Herstellung durch Umesterung von 3b-B-14 in Gegenwart von $Ti(OC_4H_9)_4$. Reinigung durch Umkristallisation aus Methylenchlorid/Ether.) |

Fortsetzung Tabelle 3b

| Verb. Nr. | n | R | Schmelzpunkt | Ausbeute [% d.Theorie] |
|---|---|---|---|---|
| 3b-B-16 | 0 | 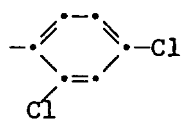 | 245°C | 85,0 (Dimethylacetamid-Zusatz. Produkt liegt als Hydrat vor (0,25 Mol $H_2O$ pro Mol Melamin).) |
| 3b-B-17 | 0 | 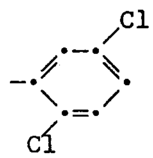 | 234°C | 80,1 (Dimethylacetamid-Zusatz.) |
| 3b-B-18 | 0 | 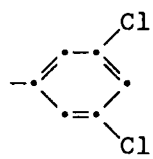 | 228°C | 86,2 (Dimethylacetamid-Zusatz.) |
| 3b-B-19 | 0 | 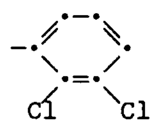 | 229°C | 86,5 (Dimethylacetamid-Zusatz.) |
| 3b-B-20 | 0 | 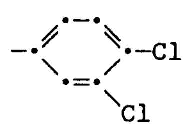 | 201°C | 81,3 (Dimethylacetamid-Zusatz.) |

Tabelle 3c:

| Verb. Nr. | n | R | Schmelzpunkt | Ausbeute [% d.Theorie] |
|---|---|---|---|---|
| 3c-B-1 | 0 | 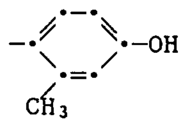 | 257°C | 61 (Produkt liegt als Hydrat vor (0,7 Mol $H_2O$ pro Mol Melamin).) |
| 3c-B-2 | 0 | 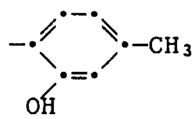 | 255°C | ~ 80 (Produkt liegt als Hydrat vor (0,15 Mol $H_2O$ pro Mol Melamin).) |
| 3c-B-3 | 0 | 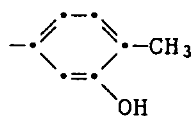 | 232°C | 69,0 (Produkt liegt als Hydrat vor (0,25 Mol $H_2O$ pro Mol Melamin).) |
| 3c-B-4 | 0 | 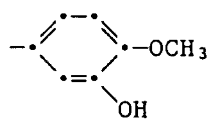 | 240°C | ~ 70 (Produkt liegt als Hydrat vor (0,25 Mol $H_2O$ pro Mol Melamin).) |
| 3c-B-5 | 0 | 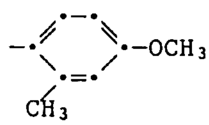 | 262°C | 75,2 (Produkt liegt als Hydrat vor (0,1 Mol $H_2O$ pro Mol Melamin).) |
| 3c-B-6 | 0 | 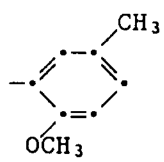 | 268°C | 89,3 (Produkt liegt als Hydrat vor (0,15 Mol $H_2O$ pro Mol Melamin).) |

Tabelle 3d:

| Verb. Nr. | n | R | Schmelzpunkt | Ausbeute [% d.Theorie] |
|---|---|---|---|---|
| 3d-B-1 | 0 | 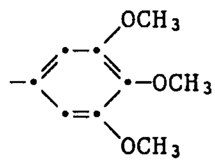 | 230°C (Nach Reinigung über das Hydrochlorid 210°C.) | 84,3 (Produkt liegt als Hemihydrat vor.) |
| 3d-B-2 | 1 | 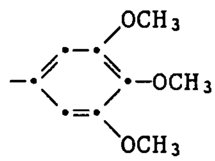 | 258°C | 77,4 (Hergestellt durch Behandeln von 3d-B-1 mit HCl. Umkristallisation aus Wasser mit Aktivkohle. Produkt liegt als Hydrat vor (0,75 Mol $H_2O$ pro Mol Melamin).) |
| 3d-B-3 | 0 | 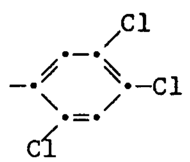 | 200°C | 30,1 (Dimethylacetamid-Zusatz. Reinigung durch Umfällen mit Dimethylsulfoxid/ $H_2O$.) |
| 3d-B-4 | 0 | 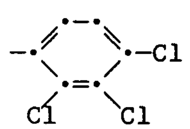 | 150°C | 22,1 (Dimethylacetamid-Zusatz. Reinigung durch Umfällen mit Dimethylsulfoxid/ $H_2O$.) |
| 3d-B-5 | 0 | 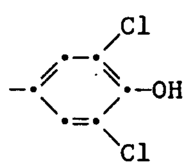 | > 300°C | 91,7 (Produkt liegt als Hydrat vor (0,4 Mol $H_2O$ pro Mol Melamin).) |

Beispiel 9:

Eine Trockenmischung bestehend aus 100 Teilen S-PVC (®Vinnol H 70 DF), 17 Teilen Dioctylphthalat, 3 Teilen epoxidiertem Sojabohnenöl, 0,33 Teilen Zn-Oleat, 0,53 Teilen Ba-p-(t-butyl)benzoat, 0,7 Teilen Diisodecyl-phenylphosphit, 0,44 Teilen ®SHELL SOL A (aromatisches Kohlenwasserstoff-Gemisch) und 0,2 Teilen des in den Tabellen 4a bis 4h angegebenen Melaminderivats wird auf einem Mischwalzwerk 5 Minuten bei 190°C gewalzt. Vom gebildeten 0,3 mm dicken Walzfell werden Folienmuster in einem Testofen (®Mathis-Thermotester) bei 180°C thermisch belastet. Im angegebenen Zeitintervall wird an einem Prüfmuster der "Yellowness Index" (YI) nach ASTM D 1925 bestimmt. Die Ergebnisse sind in den Tabellen 4a bis 4h aufgeführt.

Tabelle 4a:

| Melaminderivat | YI nach Belastungszeit in Minuten | | | | |
|---|---|---|---|---|---|
| | 0 | 5 | 10 | 15 | 20 |
| ohne | 9,2 | 17,6 | 16,8 | 15,7 | 13,8 |
| Verbindung 2a-A-4 | 4,1 | 5,1 | 5,2 | 5,3 | 6,3 |
| Verbindung 3a-B-14 | 2,0 | 8,1 | 7,7 | 7,8 | 8,3 |

Tabelle 4b:

| Melaminderivat | YI nach Belastungszeit in Minuten | | | | |
|---|---|---|---|---|---|
| | 0 | 5 | 10 | 15 | 20 |
| ohne | 10,4 | 14,4 | 15,4 | 16,6 | 16,8 |
| Verbindung 1-A-6 | 5,5 | 6,7 | 6,6 | 7,7 | 9,0 |

Tabelle 4c:

| Melaminderivat | YI nach Belastungszeit in Minuten | | | | |
|---|---|---|---|---|---|
| | 0 | 5 | 10 | 15 | 20 |
| ohne | 9,7 | 12,3 | 14,6 | 16,3 | 17,0 |
| Verbindung 3a-B-18 | 5,7 | 7,7 | 7,9 | 7,7 | 8,2 |
| Verbindung 3a-B-19 | 5,5 | 6,5 | 6,5 | 7,0 | 7,4 |
| Verbindung 3a-B-20 | 5,3 | 6,0 | 6,5 | 7,0 | 7,3 |

Tabelle 4d

| Melaminderivat | YI nach Belastungszeit in Minuten | | | | |
|---|---|---|---|---|---|
| | 0 | 5 | 10 | 15 | 20 |
| ohne | 9,8 | 12,0 | 13,5 | 15,8 | 17,1 |
| Verbindung 3a-B-1 | 5,7 | 6,4 | 7,4 | 7,5 | 7,8 |
| Verbindung 3a-B-2 | 5,4 | 6,5 | 6,7 | 7,0 | 7,3 |
| Verbindung 3a-B-3 | 5,3 | 7,0 | 7,5 | 7,4 | 7,6 |
| Verbindung 3a-B-4 | 5,2 | 6,3 | 6,6 | 7,0 | 7,0 |
| Verbindung 3a-B-5 | 5,6 | 7,3 | 7,3 | 7,6 | 8,3 |

Tabelle 4e

| Melaminderivat | YI nach Belastungszeit in Minuten | | | | |
|---|---|---|---|---|---|
| | 0 | 5 | 10 | 15 | 20 |
| ohne | 9,0 | 14,8 | 16,7 | 17,4 | 17,1 |
| Verbindung 3b-B-1 | 3,3 | 4,5 | 5,0 | 5,5 | 6,5 |
| Verbindung 3b-B-2 | 3,2 | 4,8 | 4,7 | 5,1 | 5,6 |
| Verbindung 3b-B-3 | 2,9 | 4,4 | 5,0 | 5,2 | 5,8 |
| Verbindung 3b-B-4 | 3,3 | 3,6 | 4,2 | 4,6 | 5,5 |
| Verbindung 3b-B-5 | 2,7 | 3,5 | 3,7 | 4,7 | 4,9 |

Tabelle 4f

| Melaminderivat | YI nach Belastungszeit in Minuten | | | | |
|---|---|---|---|---|---|
| | 0 | 5 | 10 | 15 | 20 |
| ohne | 8,4 | 13,8 | 15,0 | 16,3 | 16,5 |
| Verbindung 1-A-5 | 3,0 | 4,4 | 4,6 | 4,9 | 5,9 |

Tabelle 4g

| Melaminderivat | YI nach Belastungszeit in Minuten | | | | |
|---|---|---|---|---|---|
| | 0 | 5 | 10 | 15 | 20 |
| ohne | 8,3 | 13,7 | 15,1 | 17,6 | 17,9 |
| Verbindung 3a-B-8 | 3,1 | 4,3 | 4,6 | 5,0 | 5,8 |
| Verbindung 3a-B-10 | 3,0 | 5,1 | 6,0 | 6,1 | 6,3 |
| Verbindung 3a-B-11 | 4,3 | 5,2 | 5,7 | 6,6 | 7,7 |
| Verbindung 3b-B-14 | 3,8 | 5,6 | 6,2 | 6,7 | 8,2 |
| Verbindung 3b-B-15 | 4,3 | 5,7 | 5,9 | 7,1 | 7,6 |

Tabelle 4h

| Melaminderivat | YI nach Belastungszeit in Minuten | | | | |
|---|---|---|---|---|---|
| | 0 | 5 | 10 | 15 | 20 |
| ohne | 10,7 | 16,4 | 18,3 | 17,9 | 17,9 |
| Verbindung 3b-B-20 | 3,5 | 4,8 | 5,2 | 5,9 | 6,4 |
| Verbindung 3b-B-6 | 3,1 | 4,2 | 5,0 | 5,2 | 6,1 |

Beispiel 10:

Eine Trockenmischung bestehend aus 100 Teilen PVC (®Solvic 264 GA), 3 Teilen epoxidiertem Sojabohnenöl, 0,35 Teilen Ca-Stearat, 0,15 Teilen Zn-Stearat, 0,55 Teilen Diisodecyl-phenylphosphit und 0,3 Teilen des in den Tabellen 5a bis 5 k angegebenen Melaminderivats wird auf einem Mischwalzwerk 5 Minuten bei 180 °C gewalzt. Vom gebildeten 0,3 mm dicken Walzfell werden Folienmuster in einem Testofen (®Mathis-Thermotester) bei 180 °C thermisch belastet. Im angegebenen Zeitintervall wird an einem Prüfmuster der "Yellowness Index" (YI) nach ASTM D 1925 bestimmt. Die Ergebnisse sind in den Tabellen 5a bis 5k aufgeführt.

Tabelle 5a:

| Melaminderivat | YI nach Belastungszeit in Minuten | | | | | | |
|---|---|---|---|---|---|---|---|
| | 0 | 5 | 10 | 15 | 20 | 25 | 30 |
| ohne | 20,1 | 25,1 | 29,9 | 31,8 | 28,1 | 31,6 | 62,8 |
| Verbindung 2a-A-3 | 2,6 | 4,7 | 4,0 | 6,9 | 10,5 | 18,7 | 35,7 |

Tabelle 5b:

| Melaminderivat | YI nach Belastungszeit in Minuten | | | | |
|---|---|---|---|---|---|
| | 0 | 5 | 10 | 15 | 20 |
| ohne | 19,5 | 27,6 | 30,5 | 33,4 | 30,2 |
| Verbindung 1-A-8 | 3,4 | 4,9 | 5,9 | 7,6 | 12,8 |

Tabelle 5c:

| Melaminderivat | YI nach Belastungszeit in Minuten | | | | |
|---|---|---|---|---|---|
| | 0 | 5 | 10 | 15 | 20 |
| ohne | 22,4 | 37,4 | 38,5 | 36,9 | 41,2 |
| Verbindung 3a-B-19 | 2,7 | 7,1 | 10,3 | 14,7 | 24,5 |
| Verbindung 3a-B-20 | 3,2 | 8,3 | 10,6 | 15,0 | 24,7 |

Tabelle 5d

| Melaminderivat | YI nach Belastungszeit in Minuten | | | | |
|---|---|---|---|---|---|
| | 0 | 5 | 10 | 15 | 20 |
| ohne | 16,9 | 36,6 | 36,4 | 34,9 | 53,3 |
| Verbindung 3b-B-5 | 2,5 | 10,4 | 12,8 | 16,6 | 26,6 |

Tabelle 5e

| Melaminderivat | YI nach Belastungszeit in Minuten | | | | |
|---|---|---|---|---|---|
| | 0 | 5 | 10 | 15 | 20 |
| ohne | 17,5 | 36,2 | 36,8 | 34,5 | 33,3 |
| Verbindung 3d-B-1 | 1,9 | 4,8 | 6,8 | 10,0 | 15,8 |

Tabelle 5f

| Melaminderivat | YI nach Belastungszeit in Minuten | | | | |
|---|---|---|---|---|---|
| | 0 | 5 | 10 | 15 | 20 |
| ohne | 18,0 | 38,3 | 38,5 | 34,7 | 37,0 |
| Verbindung 2c-A-1 | 3,6 | 6,1 | 8,8 | 13,3 | 18,2 |

Tabelle 5g

| Melaminderivat | YI nach Belastungszeit in Minuten | | | | |
|---|---|---|---|---|---|
| | 0 | 5 | 10 | 15 | 20 |
| ohne | 16,3 | 37,8 | 38,2 | 34,2 | 44,7 |
| Verbindung 3a-B-12 | 2,8 | 7,0 | 9,4 | 14,0 | 23,7 |

Tabelle 5h

| Melaminderivat | YI nach Belastungszeit in Minuten | | | | |
|---|---|---|---|---|---|
| | 0 | 5 | 10 | 15 | 20 |
| ohne | 17,5 | 36,3 | 37,4 | 57,2 | 79,0 |
| Verbindung 2a-A-4 | 3,2 | 9,2 | 11,8 | 16,6 | 21,8 |
| Verbindung 3a-B-15 | 3,1 | 11,1 | 13,1 | 17,6 | 24,1 |

Tabelle 5i

| Melaminderivat | YI nach Belastungszeit in Minuten | | | | |
|---|---|---|---|---|---|
| | 0 | 5 | 10 | 15 | 20 |
| ohne | 15,7 | 36,2 | 37,3 | 49,3 | 74,0 |
| Verbindung 3a-B-13 | 6,2 | 11,4 | 12,6 | 15,6 | 20,6 |

Tabelle 5j

| Melaminderivat | YI nach Belastungszeit in Minuten | | | | |
|---|---|---|---|---|---|
| | 0 | 5 | 10 | 15 | 20 |
| ohne | 17,0 | 38,4 | 37,5 | 33,8 | 44,5 |
| Verbindung 3b-B-11 | 2,1 | 6,6 | 8,5 | 10,6 | 12,8 |

Tabelle 5k

| Melaminderivat | YI nach Belastungszeit in Minuten | | | | |
|---|---|---|---|---|---|
| | 0 | 5 | 10 | 15 | 20 |
| ohne | 15,0 | 32,1 | 37,7 | 36,9 | 31,8 |
| Verbindung 3b-B-20 | 1,5 | 5,0 | 5,7 | 7,4 | 11,9 |

Beispiel 11:

Eine Trockenmischung bestehend aus 100 Teilen S-PVC (®Vinnol H 70 DF), 17 Teilen Dioctylphthalat, 3 Teilen epoxidiertem Sojabohnenöl, 0,33 Teilen Zn-Oleat, 0,53 Teilen Ba-p-(t-butyl)benzoat, 0,7 Teilen Diisodecyl-phenylphosphit, 0,44 Teilen Butyldiglykol und 0,2 Teilen des in Tabelle 6 angegebenen Melaminderivats wird auf einem Mischwalzwerk 5 Minuten bei 190 °C gewalzt. Vom gebildeten 0,3 mm dicken Walzfell werden Folienmuster in einem Testofen (®Mathis-Thermotester) bei 180 °C thermisch belastet. Im angegebenen Zeitintervall wird an einem Prüfmuster der "Yellowness Index" (YI) nach ASTM D 1925 bestimmt. Die Ergebnisse sind in Tabelle 6 aufgeführt.

Tabelle 6:

| Melaminderivat | YI nach Belastungszeit in Minuten | | | | | | | | | |
|---|---|---|---|---|---|---|---|---|---|---|
| | 0 | 5 | 10 | 15 | 20 | 25 | 30 | 35 | 40 | 45 |
| ohne | 9,2 | 11,6 | 13,6 | 15,2 | 18,5 | 18,7 | 17,4 | 15,9 | 14,6 | 15,1 |
| Verbindung 2a-A-3 | 2,8 | 3,0 | 3,2 | 3,7 | 3,8 | 4,3 | 5,2 | 5,1 | 5,5 | 7,7 |

## Patentansprüche

1. Zusammensetzungen enthaltend
   a) ein chlorhaltiges Polymerisat,
   b) 0,01 bis 0,9 Gew.%, bezogen auf das chlorhaltige Polymerisat, einer Verbindung der Formel I und/oder deren Hydrochlorids,

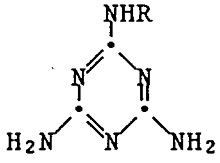

(I)

worin R $C_6$-$C_{20}$-Alkyl, durch 1 bis 5 Sauerstoffatome unterbrochenes $C_3$-$C_{20}$-Alkyl, durch 1 bis 5 OH substituiertes $C_1$-$C_{20}$-Alkyl, $C_3$-$C_8$-Cycloalkyl, durch $C_1$-$C_4$-Alkyl substituiertes $C_3$-$C_8$-Cycloalkyl, $C_7$-$C_{11}$-Phenylalkyl, am Phenyl durch 1 bis 3 Reste substituiertes $C_7$-$C_{11}$-Phenylalkyl, wobei die Reste unabhängig voneinander Hydroxy, Chlor, $C_1$-$C_4$-Alkyl, Methoxy oder Ethoxy bedeuten, oder R ferner eine Gruppe der Formel IIa oder IIb ist,

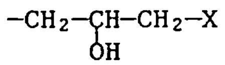 ,

(IIa)

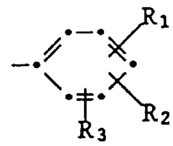

(IIb)

worin X $C_1$-$C_{20}$-Alkyloxy, $C_1$-$C_{20}$-Alkylthio, Phenyloxy, Phenylthio, Benzyloxy oder Benzylthio bedeutet und $R_1$, $R_2$ und $R_3$ unabhängig voneinander Wasserstoff, $C_1$-$C_{20}$-Alkyl, $C_1$-$C_{20}$-Alkyloxy, $C_1$-$C_{20}$-Alkylthio, $C_2$-$C_{12}$-Alkyloxycarbonyl, $C_2$-$C_{12}$-Alkanoyl, Phenyl, Phenyloxy, Phenylthio, Hydroxy, Mercapto oder Chlor sind, und

c) 0,01 bis 5 Gew.%, bezogen auf das chlorhaltige Polymerisat, eines Me(II)-Carboxylats und/oder Me(II)-Phenolats, wobei Me(II) Ba, Ca, Mg, Cd oder Zn bedeutet.

**2.** Zusammensetzungen gemäss Anspruch 1 mit der Bedingung, dass die Komponente c) ein Gemisch aus Ba/Zn-Carboxylaten darstellt, wenn die Komponente b) Phenylmelamin ist.

**3.** Zusammensetzungen gemäss Anspruch 1, worin R $C_{10}$-$C_{18}$-Alkyl, durch OH substituiertes $C_1$-$C_{10}$-Alkyl, $C_5$-$C_7$-Cycloalkyl, durch $C_1$-$C_4$-Alkyl substituiertes $C_5$-$C_7$-Cycloalkyl, Benzyl, am Phenyl durch 1 bis 3 Reste substituiertes Benzyl, wobei die Reste unabhängig voneinander Hydroxy, Chlor, $C_1$-$C_4$-Alkyl, Methoxy oder Ethoxy bedeuten, oder R ferner eine Gruppe der Formel IIa oder IIb ist, worin X $C_1$-$C_{10}$-Alkyloxy, $C_8$-$C_{18}$-Alkylthio, Phenyloxy, Phenylthio, Benzyloxy oder Benzylthio bedeutet und $R_1$, $R_2$ und $R_3$ unabhängig voneinander Wasserstoff, $C_1$-$C_{10}$-Alkyl, $C_1$-$C_{10}$-Alkyloxy, $C_8$-$C_{18}$-Alkylthio, $C_2$-$C_{12}$-Alkyloxycarbonyl, $C_2$-$C_6$-Alkanoyl, Phenyloxy, Phenylthio, Hydroxy, Mercapto oder Chlor sind.

**4.** Zusammensetzungen gemäss Anspruch 1, worin R $C_{10}$-$C_{18}$-Alkyl, durch OH substituiertes $C_1$-$C_6$-Alkyl, Cyclohexyl oder Benzyl bedeutet.

**5.** Zusammensetzungen gemäss Anspruch 1, worin R eine Gruppe der Formel IIb ist.

**6.** Zusammensetzungen gemäss Anspruch 1, worin R eine Gruppe der Formel IIb ist und die Reste $R_1$, $R_2$ und $R_3$ unabhängig voneinander Wasserstoff, $C_1$-$C_4$-Alkyl, $C_1$-$C_4$-Alkyloxy, $C_8$-$C_{16}$-Alkylthio, $C_2$-$C_{12}$-Alkyloxycarbonyl, Hydroxy, Mercapto oder Chlor bedeuten.

**7.** Zusammensetzungen gemäss Anspruch 1, worin R eine Gruppe der Formel IIb ist, $R_1$ Wasserstoff bedeutet, $R_2$ und $R_3$ unabhängig voneinander $C_1$-$C_4$-Alkyl, $C_1$-$C_4$-Alkyloxy, $C_8$-$C_{16}$-Alkylthio; $C_2$-$C_{12}$-Alkyloxycarbonyl, Hydroxy oder Chlor bedeuten und $R_2$ zusätzlich Wasserstoff ist.

**8.** Zusammensetzungen gemäss Anspruch 1, worin R eine Gruppe der Formel IIb darstellt, $R_1$ und $R_2$ Wasserstoff sind und $R_3$ $C_1$-$C_4$-Alkyl, $C_1$-$C_4$-Alkyloxy, $C_8$-$C_{16}$-Alkylthio, $C_2$-$C_{12}$-Alkyloxycarbonyl, Hydroxy oder Chlor bedeutet.

**9.** Zusammensetzungen gemäss Anspruch 1, worin die Komponente b) das Hydrochlorid einer Verbindung der Formel I ist.

**10.** Zusammensetzungen gemäss Anspruch 1, worin die Komponente c) ein Me(II)-Carboxylat ist, wobei Me(II) Ba, Ca, Mg, Cd oder Zn bedeutet.

**11.** Zusammensetzungen gemäss Anspruch 1, worin die Komponente c) ein Gemisch aus Ba/Zn- und/oder Ca/Zn-Carboxylaten ist.

**12.** Zusammensetzungen gemäss Anspruch 1, worin die Komponente a) Polyvinylchlorid ist.

**13.** Verwendung eines Stabilisatorsystems enthaltend eine Menge der Komponenten b) und c) gemäss Anspruch 1 zum Stabilisieren eines chlorhaltigen Polymerisats gegen thermischen Abbau.

## Claims

**1.** A composition comprising

a) a chlorine-containing addition polymer,

b) 0.01 to 0.9% by weight, relative to the chlorine-containing addition polymer, of a compound of the formula I and/or its hydrochloride,

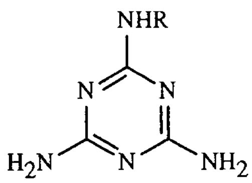

(I)

in which R is $C_6$-$C_{20}$alkyl, $C_3$-$C_{20}$alkyl interrupted by 1 to 5 oxygen atoms, $C_1$-$C_{20}$alkyl substituted by 1 to 5 OH, $C_3$-$C_8$cycloalkyl, $C_1$-$C_4$alkyl-substituted $C_3$-$C_8$cycloalkyl, $C_7$-$C_{11}$phenylalkyl, $C_7$-$C_{11}$phenylalkyl which is substituted on the phenyl by 1 to 3 radicals, these radicals, independently of one another, being hydroxyl, chlorine, $C_1$-$C_4$alkyl, methoxy or ethoxy, or R is furthermore a group of the formula IIa or IIb,

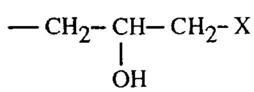 ,

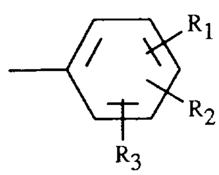

(IIa) (IIb)

in which X is $C_1$-$C_{20}$alkyloxy, $C_1$-$C_{20}$alkylthio, phenyloxy, phenylthio, benzyloxy or benzylthio, and $R_1$, $R_2$ and $R_3$, independently of one another, are hydrogen, $C_1$-$C_{20}$alkyl,$C_1$-$C_{20}$alkyloxy, $C_1$-$C_{20}$alkylthio, $C_2$-$C_{12}$alkyloxycarbonyl, $C_2$-$C_{12}$alkanoyl, phenyl, phenyloxy, phenylthio, hydroxyl, mercapto or chlorine, and

c) 0.01 to 5% by weight, relative to the chlorine-containing addition polymer, of an Me(II) carboxylate and/or Me(II)-phenolate, in which Me(II) is Ba, Ca, Mg, Cd or Zn.

**2.** A composition according to claim 1 with the proviso that component c) is a mixture of barium/zinc carboxylates if component b) is phenylmelamine.

**3.** A composition according to claim 1, in which R is $C_{10}$-$C_{18}$alkyl, OH-substituted $C_1$-$C_{10}$alkyl, $C_5$-$C_7$cycloalkyl, $C_1$-$C_4$alkyl-substituted $C_5$-$C_7$cycloalkyl, benzyl, benzyl which is substituted on the phenyl by 1 to 3 radicals, these radicals, independently of one another, being hydroxyl, chlorine, $C_1$-$C_4$alkyl, methoxy or ethoxy, or R is furthermore a group of the formula IIa or IIb, in which X is $C_1$-$C_{10}$alkyloxy, $C_8$-$C_{18}$alkylthio, phenyloxy, phenylthio, benzyloxy or benzylthio and $R_1$, $R_2$ and $R_3$, independently of one another, are hydrogen, $C_1$-$C_{10}$alkyl, $C_1$-$C_{10}$alkyloxy, $C_8$-$C_{18}$alkylthio, $C_2$-$C_{12}$alkyloxycarbonyl, $C_2$-$C_6$alkanoyl, phenyloxy, phenylthio, hydroxyl, mercapto or chlorine.

**4.** A composition according to claim 1, in which R is $C_{10}$-$C_{18}$alkyl, OH-substituted $C_1$-$C_6$alkyl, cyclohexyl or benzyl.

**5.** A composition according to claim 1, in which R is a group of the formula IIb.

**6.** A composition according to claim 1, in which R is a group of the formula IIb and the radicals $R_1$, $R_2$ and $R_3$, independently of one another, are hydrogen, $C_1$-$C_4$alkyl, $C_1$-$C_4$alkyloxy, $C_8$-$C_{16}$alkylthio, $C_2$-$C_{12}$alkyloxycarbonyl, hydroxyl, mercapto or chlorine.

**7.** A composition according to claim 1, in which R is a group of the formula IIb, $R_1$ is hydrogen, $R_2$ and $R_3$, independently of one another, are $C_1$-$C_4$alkyl, $C_1$-$C_4$alkyloxy, $C_8$-$C_{16}$alkylthio, $C_2$-$C_{12}$alkyloxycarbonyl, hydroxyl or chlorine, and $R_2$ is additionally hydrogen.

**8.** A composition according to claim 1, in which R is a group of the formula IIb, $R_1$ and $R_2$ are hydrogen and $R_3$ is $C_1$-$C_4$alkyl, $C_1$-$C_4$alkyloxy, $C_8$-$C_{16}$alkylthio, $C_2$-$C_{12}$alkyloxycarbonyl, hydroxyl or chlorine.

**9.** A composition according to claim 1, in which component b) is the hydrochloride of a compound of the formula I.

**10.** A composition according to claim 1, in which component c) is an Me(II) carboxylate, in which Me(II) is Ba, Ca, Mg, Cd or Zn.

**11.** A composition according to claim 1, in which component c) is a mixture of barium/zinc carboxylates and/or calcium/zinc carboxylates.

**12.** A composition according to claim 1, in which component a) is polyvinyl chloride.

**13.** The use of a stabiliser system comprising a quantity of components b) and c) according to claim 1 for stabilising a chlorine-containing addition polymer against thermal degradation.

**Revendications**

**1.** Compositions contenant
a) un polymère chloré,
b) 0,01 à 0,9 % en masse, par rapport au polymère chloré, d'un composé de formule I et/ou de son chlorhydrate

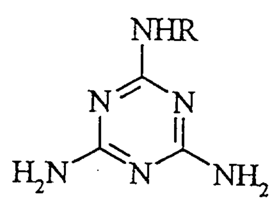

(I)

où R représente un reste alkyle en $C_6$-$C_{20}$, alkyle en $C_3$-$C_{20}$ interrompu par 1 à 5 atomes d'oxygène, alkyle en $C_1$-$C_{20}$ substitué par 1 à 5 OH, cycloalkyle en $C_3$-$C_8$, cycloalkyle en $C_3$-$C_8$ substitué par alkyle en $C_1$-$C_4$, phénylalkyle en $C_7$-$C_{11}$, phénylalkyle en $C_7$-$C_{11}$ substitué sur le phényle par 1 à 3 restes qui sont indépendamment les uns des autres des restes hydroxy, chloro, alkyle en $C_1$-$C_4$, méthoxy ou éthoxy, ou bien R représente en outre un groupe de formule IIa ou IIb,

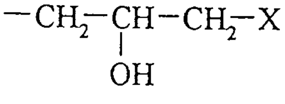

,

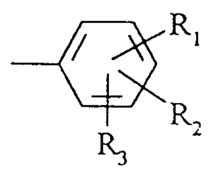

(IIa) (IIb)

où X représente un reste alcoxy en $C_1$-$C_{20}$, alkylthio en $C_1$-$C_{20}$, phényloxy, phénylthio, benzyloxy ou benzylthio et $R_1$, $R_2$ et $R_3$ sont indépendamment les uns des autres un atome d'hydrogène ou un reste alkyle en $C_1$-$C_{20}$, alcoxy en $C_1$-$C_{20}$, alkylthio en $C_1$-$C_{20}$, alcoxycarbonyle en $C_2$-$C_{12}$, alcanoyle en $C_2$-$C_{12}$, phényle, phényloxy, phénylthio, hydroxy, mercapto ou chloro, et

c) 0,01 à 5 % en masse, par rapport au polymère chloré, d'un carboxylate de Me(II) et/ou d'un phénolate de Me(II), Me(II) désignant Ba, Ca, Mg, Cd ou Zn.

**2.** Compositions selon la revendication 1, avec la condition selon laquelle le constituant c) représente un mélange de carboxylates de Ba/Zn lorsque le constituant b) est la phénylmélamine.

**3.** Compositions selon la revendication 1, dans lesquelles R est un reste alkyle en $C_{10}$-$C_{18}$, alkyle en $C_1$-$C_{10}$ substitué par OH, cycloalkyle en $C_5$-$C_7$, cycloalkyle en $C_5$-$C_7$ substitué par alkyle en $C_1$-$C_4$, benzyle, benzyle substitué sur le phényle par 1 à 3 restes qui sont indépendamment les uns des autres des restes hydroxy, chloro, alkyle en $C_1$-$C_4$, méthoxy ou éthoxy, ou bien R représente en outre un groupe de formule IIa ou IIb, où X est un reste alcoxy en $C_1$-$C_{10}$, alkylthio en $C_8$-$C_{18}$,phényloxy, phénylthio, benzyloxy ou benzylthio et $R_1$, $R_2$ et $R_3$ sont indépendamment les uns des autres des atomes d'hydrogène ou des restes alkyle en $C_1$-$C_{10}$, alcoxy en $C_1$-$C_{10}$, alkylthio en $C_8$-$C_{18}$, alcoxycarbonyle en $C_2$-$C_{12}$, alcanoyle en $C_2$-$C_6$, phényloxy, phénylthio, hydroxy, mercapto ou chloro.

**4.** Compositions selon la revendication 1, dans lesquelles R est un reste alkyle en $C_{10}$-$C_{18}$, alkyle en $C_1$-$C_6$ substitué par OH, cyclohexyle ou benzyle.

**5.** Compositions selon la revendication 1, dans lesquelles R représente le groupe de formule IIb.

**6.** Compositions selon la revendication 1, dans lesquelles R est un groupe de formule IIb et les restes $R_1$, $R_2$ et $R_3$ représentent indépendamment les uns des autres des atomes d'hydrogène ou des restes alkyle en $C_1$-$C_4$, alcoxy en $C_1$-$C_4$, alkylthio en $C_8$-$C_{16}$, alcoxycarbonyle en $C_2$-$C_{12}$, hydroxy, mercapto ou chloro.

**7.** Compositions selon la revendication 1, dans lesquelles R représente un groupe de formule IIb, $R_1$ est un hydrogène, $R_2$ et $R_3$ représentent indépendamment l'un de l'autre un reste alkyle en $C_1$-$C_4$, alcoxy en $C_1$-$C_4$, alkylthio en $C_8$-$C_{16}$, alcoxycarbonyle en $C_2$-$C_{12}$, hydroxy ou chloro, et $R_2$ peut en plus être un hydrogène.

**8.** Compositions selon la revendication 1, dans lesquelles R représente un groupe de formule IIb, $R_1$ et $R_2$ sont l'hydrogène et $R_3$ représente un reste alkyle en $C_1$-$C_4$, alcoxy en $C_1$-$C_4$, alkylthio en $C_8$-$C_{16}$, alcoxycarbonyle en $C_2$-$C_{12}$, hydroxy ou chloro.

**9.** Compositions selon la revendication 1, dans lesquelles le constituant b) est le chlorhydrate d'un composé de formule I.

**10.** Compositions selon la revendication 1, dans laquelle le constituant c) est un carboxylate de Me(II), Me(II) représentant Ba, Ca, Mg, Cd ou Zn.

**11.** Compositions selon la revendication 1, dans lesquelles le constituant c) est un mélange de carboxylates de Ba/Zn et/ou de Ca/Zn.

**12.** Compositions selon la revendication 1, dans lesquelles le constituant a) est du poly(chlorure de vinyle).

**13.** Utilisation d'un système stabilisant contenant une quantité des constituants b) et c) selon la revendication 1 pour la stabilisation d'un polymère chloré contre la dégradation thermique.